(19) Europäisches Patentamt European Patent Office Office européen des brevets

(11) **EP 4 292 614 B1**

(12) **EUROPEAN PATENT SPECIFICATION**

(45) Date of publication and mention
of the grant of the patent:
**08.10.2025 Bulletin 2025/41**

(21) Application number: **22771043.1**

(22) Date of filing: **24.02.2022**

(51) International Patent Classification (IPC):
***A61L 2/10*** (2006.01)      ***C02F 1/32*** (2023.01)

(52) Cooperative Patent Classification (CPC):
**C02F 1/325; A61L 2/10;** C02F 2201/3222;
C02F 2201/3228

(86) International application number:
**PCT/JP2022/007749**

(87) International publication number:
**WO 2022/196287 (22.09.2022 Gazette 2022/38)**

(54) **FLUID STERILIZATION DEVICE**

**FLÜSSIGKEITSSTERILISATIONSVORRICHTUNG**

**DISPOSITIF DE STÉRILISATION DE FLUIDE**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.03.2021 JP 2021046358**

(43) Date of publication of application:
**20.12.2023 Bulletin 2023/51**

(73) Proprietor: **STANLEY ELECTRIC CO., LTD.**
**Tokyo 153-8636 (JP)**

(72) Inventors:
• **TANAKA Hideaki**
**Tokyo 153-8636 (JP)**

• **KATO Hiroyuki**
**Tokyo 153-8636 (JP)**
• **SHINNO Kazuhisa**
**Tokyo 153-8636 (JP)**

(74) Representative: **Schmidbauer, Andreas Konrad**
**Wagner & Geyer Partnerschaft mbB**
**Patent- und Rechtsanwälte**
**Gewürzmühlstrasse 5**
**80538 München (DE)**

(56) References cited:
WO-A1-2021/020536      CN-A- 108 793 532
JP-A- 2019 141 292      JP-A- 2021 028 056
US-A1- 2004 046 127      US-A1- 2011 024 365

## Description

Technical Field

The present invention relates to a fluid sterilization device that sterilizes a fluid flowing through a tubular passage by irradiating the fluid with ultraviolet light.

Background Art

JP 2020-92856 A discloses a fluid sterilization device that sterilizes a fluid flowing through a tubular passage by irradiating the fluid with ultraviolet light. In this fluid sterilization device, a flow-regulating plate having a plurality of through holes is disposed on the inflow port side of the tubular passage, so that the fluid flows through a sterilization space in a regulated state, thereby suppressing unevenness in the amount of ultraviolet light irradiation to the fluid flowing through the tubular passage.

CN 108 793 532 A discloses a filter and disinfection device for a cultural water body, wherein the filter and disinfection device comprises a box body; one side of the box body is provided with a cultural water body inlet pipe; an inner cavity of the box body is provided with a filter screen; the filter screen is arranged at the inner side of a water inlet of box body; the inner cavity of the box body is also provided with a filter plate, the other side of the box body is provided with a drainage port, and the drainage port communicates with a UV disinfector through a drainage pipe; the UV disinfector is connected with a discharge pipe. The device also comprises an aeration device; the aeration device is arranged above the box body, and the aeration device is connected with the box body through a pipeline.

Summary of Invention

Technical Problem

In the flow-regulating plate in JP 2020-92856 A, the through holes in the central part have larger diameters than the through holes in the peripheral part in the radial direction of the flow-regulating plate. This promotes uniformity of the fluid velocity distribution in the radial direction on the downstream side of the flow-regulating plate. The through holes in the flow-regulating plate in JP 2020-92856 A are all cylindrical holes.

Meanwhile, it has been proposed to dispose a total of two flow-regulating plates, one each on the upstream side and the downstream side with an interval provided therebetween in the axial direction. In such a case, the flow-regulating plate on the upstream side has a plurality of cylindrical first through holes in a relatively central part in the radial direction, and the flow-regulating plate on the downstream side has a plurality of cylindrical second through holes, which have larger diameters than those of the first through holes, distributed over the entire surface. As a result, a fluid flowing into the tubular passage through the inflow port can be regulated over a relatively short length in the axial direction.

An object of the present invention is to provide a fluid sterilization device capable of achieving high flow regulating function by a flow-regulating plate so as to permit a further size reduction in the axial direction.

Solution to Problem

A fluid sterilization device in accordance with the present invention is provided as set forth in claim 1. Preferred embodiments of the present invention may be gathered from the dependent claims.

Advantageous Effects of Invention

According to the present invention, the through holes of the flow-regulating plate are formed of tapered holes having the diameters thereof increasing in a fluid flowing direction in the tubular passage, thus making it possible to achieve a high flow regulating function. As a result, the dimension of a fluid sterilization device in the axial direction can be reduced.

Brief Description of Drawings

FIG. 1A is a perspective view of a liquid sterilization device.
FIG. 1B is a front view of the liquid sterilization device.
FIG. 2 is a longitudinal sectional view of the liquid sterilization device.
FIG. 3 is an enlarged view of an end portion of the liquid sterilization device on the light source side.
FIG. 4A is a view of a flow-regulating plate observed in the axial direction from the other end side.
FIG. 4B is a perspective view of the flow-regulating plate observed at an angle in the axial direction from the other end side.
FIG. 5 is a view in the cross-sectional direction.
FIG. 6A is a diagram illustrating the flow velocity distribution in the liquid sterilization device in Comparative Example 1, taken along the Ac-Ac arrow cross section in FIG. 5.
FIG. 6B is a diagram illustrating the flow velocity distribution in the liquid sterilization device in Comparative Example 2, taken along the Ac-Ac arrow cross section in FIG. 5.
FIG. 6C is a diagram illustrating the flow velocity distribution in the liquid sterilization device in Comparative Example 3, taken along the Ac-Ac arrow cross section in FIG. 5.
FIG. 6D is a diagram illustrating the flow velocity distribution in the liquid sterilization device in an embodiment, taken along the Ac-Ac arrow cross section in FIG. 5.

FIG. 7A is a diagram illustrating the flow velocity distribution in the liquid sterilization device in Comparative Example 1, taken along the Bc-Bc arrow cross section in FIG. 5.

FIG. 7B is a diagram illustrating the flow velocity distribution in the liquid sterilization device in Comparative Example 2, taken along the Bc-Bc arrow cross section in FIG. 5.

FIG. 7C is a diagram illustrating the flow velocity distribution in the liquid sterilization device in Comparative Example 3, taken along the Bc-Bc arrow cross section in FIG. 5.

FIG. 7D is a diagram illustrating the flow velocity distribution in the liquid sterilization device in the embodiment, taken along the Bc-Bc arrow cross section in FIG. 5.

FIG. 8 is a view of another flow-regulating plate observed in the axial direction from the other end side.

FIG. 9 is an explanatory diagram illustrating a preferred range of taper angles of tapered holes.

Description of Embodiments

The following will describe a plurality of embodiments of the present invention. It is needless to say that the present invention is not limited to these embodiments. The same reference numerals are used for components that are common among a plurality of embodiments, and the descriptions of components with the same reference numerals that have already been described in a preceding embodiment will be omitted in a subsequent embodiment.

(Configuration)

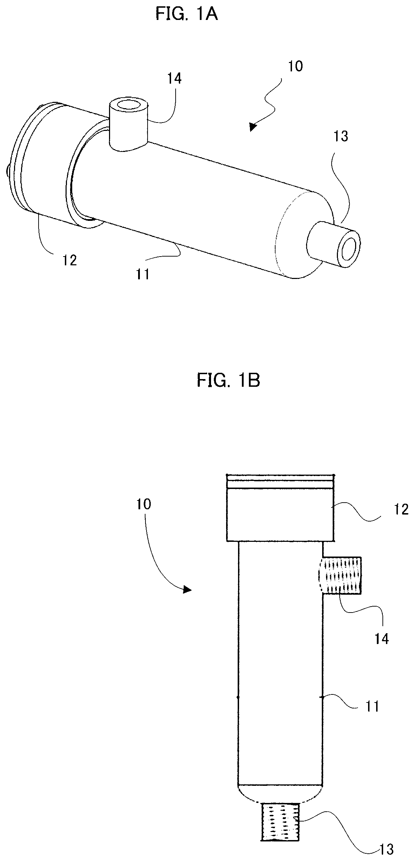

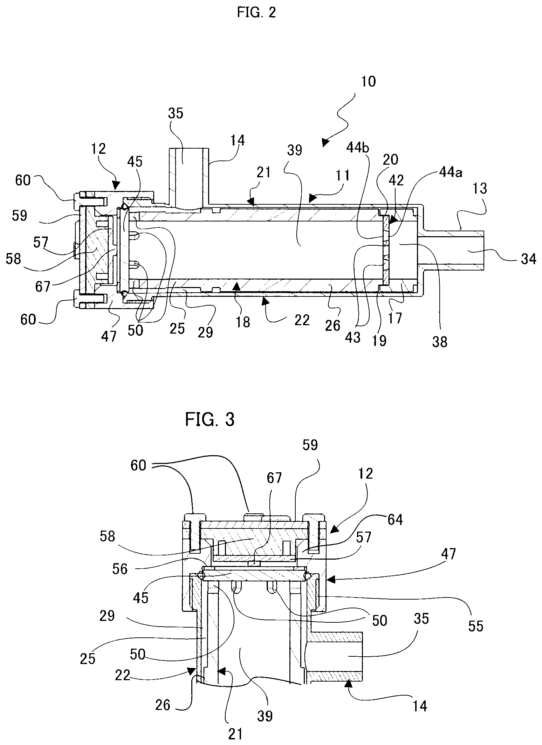

FIG. 1A is a perspective view of a liquid sterilization device 10. FIG. 1B is a front view of the liquid sterilization device 10. FIG. 2 is a longitudinal sectional view of the liquid sterilization device 10. FIG. 3 is an enlarged view of an end portion of the liquid sterilization device 10 on a light source 12 side.

The liquid sterilization device 10 includes a housing 11, the light source 12, a lead-in port section 13, and a lead-out port section 14. The lead-in port section 13 and the lead-out port section 14 are formed integrally with the housing 11. The liquid sterilization device 10 is typically placed with the axial direction thereof aligned with a vertical direction, the light source 12 being on the upper side (vertical placement), as illustrated in FIG. 1B.

The liquid sterilization device 10 is an example of a fluid sterilization device, and sterilizes water as a liquid, which is also a fluid. The liquid sterilization device 10 is installed to, for example, a water storage tank of an ice machine or the like, a water pipe, a water heater, a water server, a circulation device (chiller cooling water), and a drink server.

Water sterilized by the liquid sterilization device 10 is usually used for drinking. Sterilization in a circulation device is performed to prevent the propagation of bacteria in circulating water, which increases the viscosity of the circulating water, leading to power loss.

The lead-in port section 13 and the lead-out port section 14 are circumferentially threaded for connection to a tube (not illustrated) in an apparatus in which the liquid sterilization device 10 is to be disposed (FIG. 1B). The water to be sterilized by the ultraviolet light of the light source 12 of the liquid sterilization device 10 flows from the lead-in port section 13 side (one end side) to the lead-out port section 14 side (the other end side) in the axial direction through a tubular passage which is formed in the housing 11 and composed of a lead-in side space 38 and a sterilization space 39.

The light source 12 is attached to the housing, with the central axis thereof aligned with the central axis of the housing 11 on the other end side in the axial direction of the liquid sterilization device 10. The lead-in port section 13 is provided on an end portion on the one end side of the housing 11 in the axial direction. The lead-out port section 14 is provided on the side portion of the housing 11 in such a manner as to project in the radial direction at a position spaced away by a predetermined distance toward one end from the other end of the housing 11 in the axial direction.

The housing 11 has an inner tube 21 and an outer tube 22, which are coaxially disposed. The inner tube 21 and the outer tube 22 are both straight tubes. The lead-in port section 13 and the lead-out port section 14 are provided integrally with the outer tube 22. A lead-in port 34 and a lead-out port 35 are defined on the inner circumferential sides of the lead-in port section 13 and the lead-out port section 14, respectively, and provide communication between the inside and the outside of the outer tube 22.

The inner tube 21 has a one-end-side inner tube member 17 and an other-end-side tube member 18, which are joined to each other by the tightening force of a fixing nut 47, which will be described later, from one end side and the other end side in the axial direction. The inner tube 21 is fitted by insertion into the outer tube 22 from the opening at the other end side of the outer tube 22. The one-end-side inner tube member 17 and the other-end-side tube member 18 are open at both ends in the axial direction.

The other-end-side tube member 18 has a small-diameter portion 25 on the other end side and a large-diameter portion 26 on the one end side in the axial direction. An annular space 29 is formed between the inner circumference of the outer tube 22 and the outer circumference of the small-diameter portion 25. The boundary between the small-diameter portion 25 and the large-diameter portion 26 has a step portion. The step portion is positioned closer to the one end side than to the lead-out port 35 in the axial direction. Thus, the lead-out port 35 is entirely exposed to the annular space 29 without being covered by the large-diameter portion 26.

In this embodiment, the step portion projects from the small-diameter portion 25 in such a manner as to be perpendicular to the axial direction of the inner tube 21, i.e., parallel to the radial direction of the inner tube 21. The step portion can alternatively be a tapered step portion, in place of the vertical step portion, to ensure smooth change of direction of a water flow from the annular space 29 to the lead-out port 35.

A flow-regulating plate 42 has the circumferential edge thereof fitted by insertion into an annular groove 20 formed on the inner circumferential side of a joint portion 19 between the one-end-side inner tube member 17 and the other-end-side tube member 18, and is pinched by the mutual joining force of the one-end-side inner tube member 17 and the other-end-side tube member 18 from both sides in the axial direction. This joining force is generated by the tightening force in the axial direction of the fixing nut 47, which will be described later.

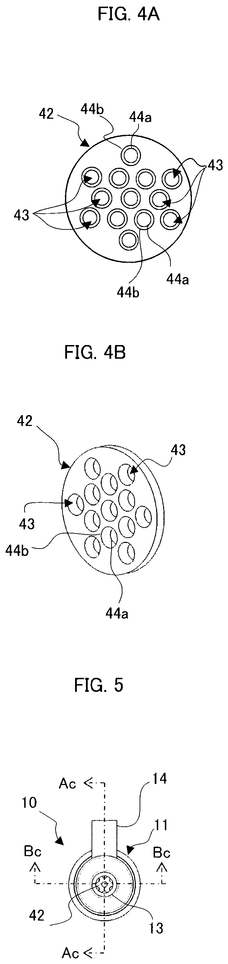

The flow-regulating plate 42 divides the tubular passage defined on the inner circumferential side by the inner tube 21 into a lead-in side space 38 on the one end side and the sterilization space 39 on the other end side. FIG. 4A and FIG. 4B illustrate the flow-regulating plate 42 observed in the axial direction from the other end side and at an angle from the other end, respectively. Referring to FIG. 2, FIG. 4A, and FIG. 4B, the flow-regulating plate 42 has a plurality of tapered holes 43 penetrating in the axial direction. The tapered holes 43 are open on both the one end side and the other end side in the axial direction through circular upstream-side openings 44a and downstream-side openings 44b. The diameters of the upstream-side openings 44a are smaller than the diameters of the downstream-side openings 44b. In other words, the diameters of the tapered holes 43 gradually increase toward the other end side from the one end side in the axial direction.

Referring to FIG. 3, the other-end-side tube member 18 has a plurality of notch grooves 50 with U-shaped cross sections provided at equal angular intervals in the circumferential direction on the circumferential end face on the other end side in the axial direction. A quartz plate 45 has a peripheral edge portion thereof on one end side fitted by insertion into the inner circumferential step portion of the other end portion of the outer tube 22 so as to be brought into contact with the circumferential end surface of the other-end-side tube member 18, and has an annular spacer 56 in contact with the circumferential edge thereof on the other end side.

A circuit board 57 is fitted by insertion into a through hole of the fixing nut 47, has a surface thereof on the quartz plate 45 side, and has a light source 67 at the center of the surface. A heat sink 58 has one end thereof inserted into the through hole of the fixing nut 47 in the axial direction, has the flange portion thereof on the other end side brought into contact with the end face of the fixing nut 47, and is fixed to the end face of the fixing nut 47 by a plurality of screws 60.

The fixing nut 47 is secured to the housing 11, which is a tubular body, by being screwed to a thread groove formed in the outer circumferential portion of the axial other end portion of the outer tube 22. This screwing moves the fixing nut 47 from the other end side to the one end side in the axial direction, and tightens, in the axial direction, the one-end-side inner tube member 17 and the other-end-side tube member 18 accommodated in the outer tube 22 to join these two tube members. Thus, the one-end-side inner tube member 17 and the other-end-side tube member 18 are joined to each other and pinch the circumferential edge of the flow-regulating plate 42 in the annular groove 20 in the axial direction.

(Bactericidal action)

Water as a fluid to be sterilized (not illustrated) is pressure-fed by a pump (not illustrated) into the lead-in port 34. Then, the water flows through the lead-in port 34 into the lead-in side space 38, where the water spreads in the radial direction of the inner tube 21 due to an increase in the passage cross-sectional area.

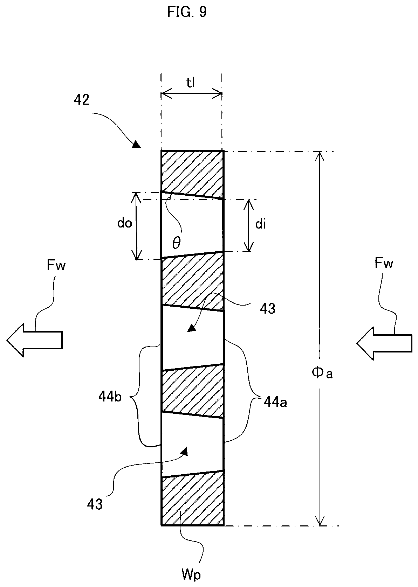

After that, the water passes through the tapered holes 43 of the flow-regulating plate 42 and enters into the sterilization space 39. When passing through the tapered holes 43, the water spreads in the radial direction, which is the vertical direction with respect to the axial direction, and spreads at a spreading angle based on the taper angle θ (FIG. 9) of the tapered holes 43 after the water is ejected through the tapered holes 43. As a result, on the other end side (downstream side) of the flow-regulating plate 42, the water streams ejected through adjacent tapered holes 43 into the sterilization space 39 come into contact with each other at an appropriate tilt angle thereby to suppress the diffusion in the radial direction, thus turning into a regulated water flow parallel to the axial direction.

The light source 67 applies ultraviolet light to water in the sterilization space 39 through the quartz plate 45 from the other end side in the axial direction. Thus, bacteria and the like mixed in the water are sterilized. The water impinges on the quartz plate 45, changes the direction thereof from the axial direction to the radially outward direction, and enters into the annular space 29 through the notch grooves 50. In the annular space 29, the water flows in the axial direction from the other end side to the one end side, which is opposite to the flow inside of the sterilization space 39, changes the flow direction thereof to the radially outward direction at the place of the lead-out port 35, and flows out of the liquid sterilization device 10 through the lead-out port 35.

The notch grooves 50 provide communication between the upper end of the sterilization space 39 and the upper end of the annular space 29, thus preventing the occurrence of stagnant air or stagnant water at the upper portions of the sterilization space 39 and the annular space 29 (the upper portion in the vertical placement in FIG. 1B).

If ultraviolet light passes through air, the intensity thereof is significantly reduced, causing a decrease in sterilizing power. Further, if water is trapped and retained in the sterilization space 39, the retained water becomes residual water in the sterilization space 39 after the operation of the liquid sterilization device 10 is finished, and bacteria propagate in the residual water if the non-operation time becomes long. Therefore, when restarting the operation, it is necessary to wait until the residual water with the propagated bacteria therein is discharged from the liquid sterilization device 10 before using sterilized water.

In the liquid sterilization device 10, the notch grooves 50 on the other end of the other-end-side tube member 18 prevent air and fluid from staying in the housing 11, thus making it possible to prevent a decrease in the sterilizing power of the liquid sterilization device 10 and the occurrence of residual water.

(Flow velocity distribution diagrams)

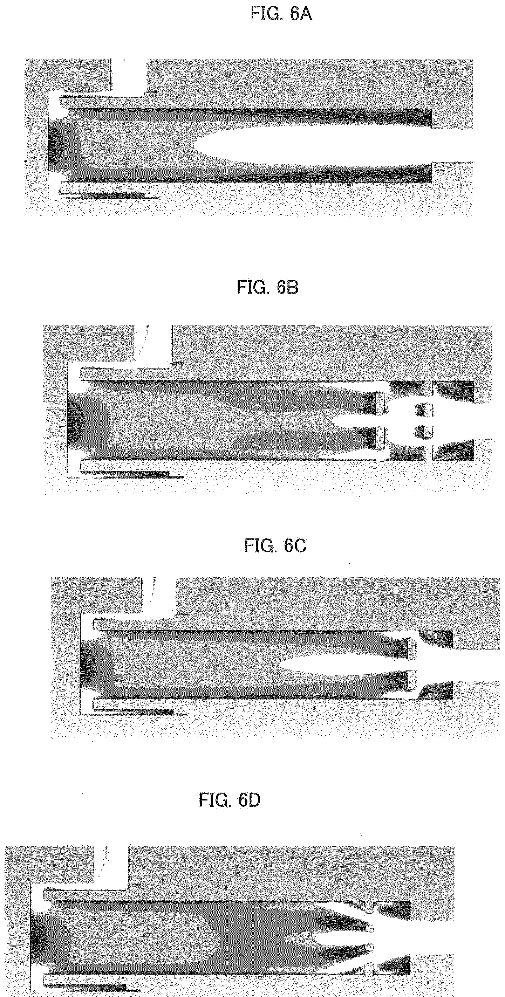

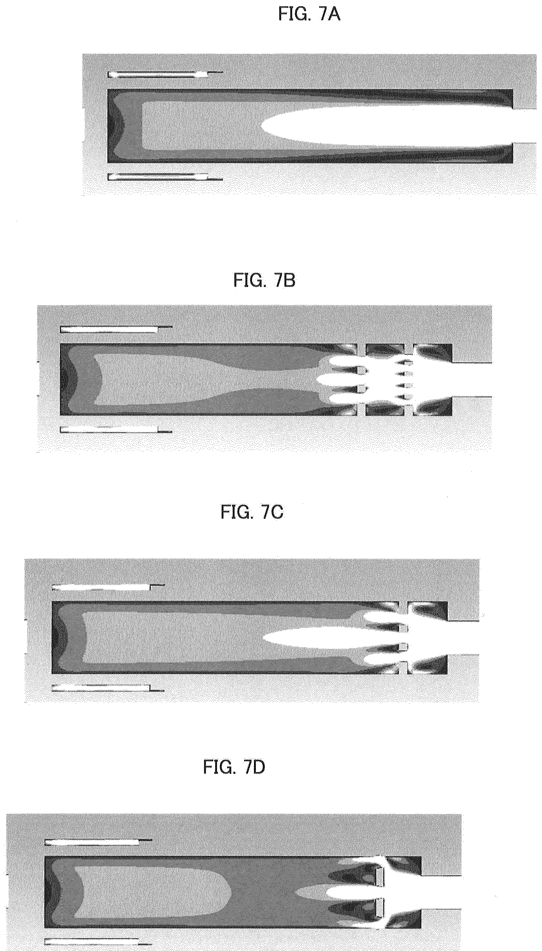

FIG. 5 is a diagram illustrating cross-sectional directions. FIG. 6A, FIG. 6B, FIG. 6C and FIG. 6D are diagrams illustrating the flow velocity distributions in the fluid sterilization devices of Comparative Examples 1, 2, and 3 and the liquid sterilization device 10 of the embodiment, respectively, taken along the Ac-Ac arrow cross section in FIG. 5. FIG. 7A, FIG. 7B, FIG. 7C and FIG. 7D are diagrams illustrating the flow velocity distributions in the fluid sterilization devices of Comparative Examples 1, 2, and 3 and the liquid sterilization device 10 of the embodiment, respectively, taken along the Bc-Bc arrow cross section in FIG. 5.

The flow velocity distributions in these diagrams have been obtained by simulations performed by the inventors, and the flow velocities are indicated in a plurality of stages. These diagrams indicate that the whiter the areas are, the higher the flow velocities are.

Comparative Example 1 corresponds to the flow velocity distribution diagram obtained when the flow-regulating plate is absent. Comparative Example 2 corresponds to the flow velocity distribution diagram obtained when two flow-regulating plates are provided. The through holes in the axial direction of the two flow-regulating plates of Comparative Example 2 are both cylindrical holes, and the diameters of the cylindrical holes of the flow-regulating plate on the upstream side are smaller than those of the flow-regulating plate on the downstream side. Comparative Example 3 is an example having one flow-regulating plate with cylindrical through holes. Meanwhile, the liquid sterilization device 10 of the embodiment in FIG. 6D and FIG. 7D is provided with the flow-regulating plate 42. The taper angle θ (FIG. 9) of the tapered holes 43 in the flow-regulating plate 42 is set to 14°.

In Comparative Examples 1 (FIG. 6A and FIG.

7A) and 3 (FIG. 6C and FIG. 7C), the white areas extend long toward the other end side, whereas in Comparative Example 2 (FIG. 6B and FIG. 7B), the white area is short. The small white area in the tubular passage means a higher level of flow regulation. It is seen that the axial position at which a fluid ejected from one flow-regulating plate in the liquid sterilization device 10 of the embodiment (FIG. 6D and 7D) reaches a stable regulated state following the ejection is substantially the axial position of the flow-regulating plate on the downstream side in Comparative Example 2 (FIG. 6B and FIG. 7B). This indicates that using the flow-regulating plate 42 of the liquid sterilization device 10 allows the liquid sterilization device 10 to be reduced in size in the axial direction while obtaining the same flow regulating effect as in the case where two flow-regulating plates having different hole diameters are used.

The present inventors have further obtained the following findings through simulation tests. In Comparative Example 2 (using the two flow-regulating plates), when the length of the flow regulating chamber (the length of the portion upstream from the flow-regulating plates) is reduced from 10 mm to 5 mm, the effect of homogenizing the flow velocity distribution weakens. In contrast, it has been found that, in the liquid sterilization device 10 of the embodiment, even when the length of the flow regulating chamber (the length of the portion upstream from the flow-regulating plate, which corresponds to the length of the one-end-side inner tube member 17) is reduced from 10 mm to 5 mm, the effect of homogenizing the flow velocity distribution can be maintained.

Further, in FIG. 6D and FIG. 7D, the taper angle θ (FIG. 9) of the tapered holes 43 was set to 14°, but even when the taper angle θ is increased to 18.4°, substantially the same effect as that in the case where the taper angle θ is set to 14° is obtained. The preferred range of the taper angle θ will be described next with reference to FIG. 9.

(Range of the taper angle θ)

FIG. 9 is an explanatory diagram illustrating the preferred range of the taper angle θ of the tapered holes 43. The taper angle θ of the tapered hole 43 is defined as the intersection angle between the side edge line of the tapered hole 43 and the central axis line of the flow-regulating plate 42 in the cross section of the flow-regulating plate 42 when the flow-regulating plate 42 is cut by a plane including the central axis line thereof (the cross section illustrated in FIG. 9). The direction of the central axis line of the flow-regulating plate 42 and the axial direction of the liquid sterilization device 10 are parallel to each other.

The definitions of the symbols in FIG. 9 are as shown below.

> t1: thickness of the flow-regulating plate 42 (= dimension of the flow-regulating plate 42 in the direction of the central axis line)

di: diameter of the upstream-side opening 44a
do: diameter of the downstream-side opening 44b
Fw: flowing direction of main flow water in tubular passage
$\phi a$: diameter of the flow-regulating plate 42
Ra: predetermined value below 1 (example: 0.65)

Expression 1 given below is introduced to set the preferred range of the taper angle $\theta$.

$$\text{Expression 1: } (2 \cdot tl) / (di + do) < Ra$$

The reason why 0.65 is desirable for Ra is as follows. A case is assumed, where di = do in the left side of Expression 1. di = do means cylindrical holes instead of the tapered holes 43. If Ra $\geq$ 0.65 is set when a fluid sterilization device includes only one flow-regulating plate, and a plurality of through holes of the only one flow-regulating plate are all cylindrical holes of equal diameters, then inappropriate flow regulation will result on the downstream side of the flow-regulating plate, i.e., poor uniformity in the flow velocity distribution in the radial direction of the tubular passage will result, leading to an inadequate function as the flow-regulating plate. For this reason, 0.65 has been selected for Ra.

The value of Ra has to be below 1, because, if Ra $\geq$ 1, then the thickness of the flow-regulating plate will be larger than the diameter of the through holes, and the through holes become orifices, leading to a significant decrease in flow velocity. The flow-regulating plate is required to have a function as a partition wall Wp (FIG. 9) in order to perform the flow regulating function.

Regarding the relationship between the taper angle $\theta$ and each parameter of Expression 1, there is the relationship of Expression 2 given below.

$$\text{Expression 2: } \tan \theta = (do - di) / (2 \cdot tl)$$

For example, if di = 3 mm, do = 4 mm, and tl = 2 mm, then Expression 1 is satisfied, and $\theta$ = 14° is obtained. Further, if di = 3mm, do = 5 mm, and tl = 2mm, then Expression 1 is satisfied, and $\theta$ = 26.5° is obtained. The taper angles $\theta$ = 14° and 18.4° described in relation to FIG. 6D and FIG. 7D mentioned above are also values within the range that satisfies the conditions of Expression 1.

The preferred range of the taper angle $\theta$ is summarized as a range of the taper angle $\theta$ which satisfies the requirements of Expression 1 and which is derived from Expression 2. Since a plurality of tapered holes 43 must be formed in the flow-regulating plate 42, do < $\phi a$ naturally applies.

(Another flow-regulating plate)

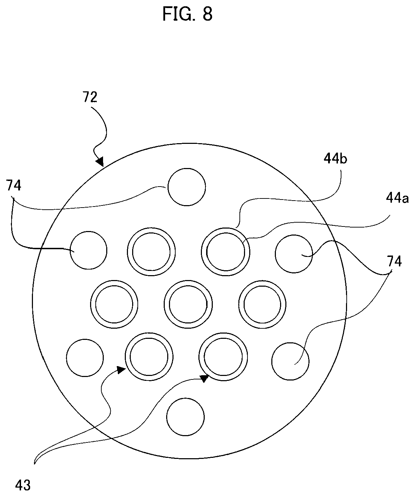

FIG. 8 is a view of another flow-regulating plate 72, which is different from the flow-regulating plate 42, observed from the other end side in the axial direction. The flow-regulating plate 72 has a plurality of tapered holes 43 (FIG. 4A) as through holes penetrating in the axial direction and a plurality of cylindrical holes 74, which are not tapered holes. When the flow-regulating plate 72 is divided into two parts, namely, a central part and a circumferential part, in the radial direction, the tapered holes 43 are formed in the central part and the cylindrical holes 74 are formed in the circumferential part.

When water as a fluid to be sterilized by a liquid sterilization device 10 flows into a lead-in side space 38 through a lead-in port 34, the water spreads in the radial direction. The water on the circumferential side of the lead-in side space 38 will proceed, with the spreading thereof in the radial direction suppressed by the inner circumferential walls of a one-end-side inner tube member 17 and the other-end-side tube member 18, until reaching notch grooves 50 in the axial direction. Conversely, if the water on the circumferential side is allowed to pass through the tapered holes 43, there is an increased possibility that the flow in the axial direction desirably running along the inner circumferential wall of the lead-in side space 38 is interfered with by the tapered holes 43.

Therefore, the flow-regulating plate 72 is designed such that the water at the radially central portion thereof passes through the tapered holes 43, while the water at the circumferential portion thereof passes through the cylindrical holes 74, thus achieving higher flow regulating effect than in the case where the water in the circumferential portion is allowed to pass through the tapered holes 43.

Further, in both flow-regulating plates 42 (FIG. 4A) and 72 (FIG. 8), the plurality of tapered holes 43 are continuous in one of the array directions (vertical, horizontal or diagonal array direction), and one tapered hole 43 is not isolated from other tapered holes 43, i.e., none of the tapered holes 43 are adjacent to the cylindrical holes 74 in any array direction.

(Modified example)

In the liquid sterilization device 10, the ultraviolet light from the circuit board 57 is applied, in the axial direction, to water, which is a fluid to be sterilized, in the sterilization space 39. A light source of ultraviolet light of the present invention may be placed outside a tube body in the radial direction such that the ultraviolet light is applied to the water in the sterilization space 39 from the radially outer side. In such a case, the liquid sterilization device 10 does not have to have a dual structure composed of the inner tube 21 and the outer tube 22, and the inner tube 21 is omitted. Further, water flows out to the lead-out port 35 directly from the sterilization space 39 without passing through the annular space 29.

In the liquid sterilization device 10, the housing 11 constitutes the tube body, and the outer tube 22 constitutes the cylindrical member. In addition, the annular space 29 is formed between the outer circumferential portion of the other-end-side tube member 18 and the inner circumferential portion of the outer tube 22. In the present invention, the annular space 29 and the notch grooves 50 can be omitted. In such a case, the lead-out port 35 opens to the sterilization space 39 in the other-end-side tube member 18 and directly communicates with the sterilization space 39 without the annular space 29 interposed therebetween.

In the liquid sterilization device 10, the quartz plate 45 is provided as a plate-like transmission member. The plate-like transmission member of the present invention can also use other materials that have a predetermined resistance to ultraviolet light, are capable of transmitting ultraviolet light, and ensure strength.

## Claims

1. A fluid sterilization device (10) comprising:

   a tube body (11) having a tubular passage through which a fluid flowing in through an inflow port (34) on one end side in an axial direction flows in the axial direction to an outflow port (35) on the other end side in the axial direction;
   a flow-regulating plate (42, 72) which divides the tubular passage into a lead-in side space (38) which is located on the one end side and into which the fluid flowing in from the inflow port (34) is introduced, and a sterilization space (39) on the other end side, and which has a plurality of through holes (43, 74) penetrating in the axial direction; and
   a light source (12, 67) which irradiates the sterilization space (39) with ultraviolet light;
   wherein the plurality of through holes (43) include a plurality of tapered holes (43) having diameters thereof increasing from the one end side toward the other end side in the axial direction,
   **characterized in that**
   each of the plurality of tapered holes (43) has an upstream side opening (44a) on the one end side and a downstream side opening (44b) on the other end side, and wherein the side wall of the tapered holes (43) forms a predetermined taper angle ($\theta$) with respect to the axial direction, and
   wherein each of the plurality of tapered holes (43) is formed so that a diameter of the upstream side opening (44a) is smaller than a diameter of the downstream side opening (44b), and the predetermined taper angle ($\theta$) is an angle with which fluids ejected into the sterilization space (39) through the tapered holes (43) which are adjacent to each other come into contact with each other to suppress diffusion in a radial direction.

2. The fluid sterilization device according to claim 1, wherein the tube body (11) has:

   a one-end-side tube member (17) which defines the lead-in side space (38) on an inner circumferential side; and
   an other-end-side tube member (18) which defines the sterilization space (39) on the inner circumferential side, which is joined to the one-end-side tube member (17) in the axial direction, and which pinches a circumferential edge of the flow-regulating plate (42, 72) in the axial direction at the inner circumference of a joint portion (19) by an annular groove (20) formed between itself and the one-end-side tube member (17).

3. The fluid sterilization device according to claim 1 or 2,

   wherein the inflow port (34) opens to the lead-in side space (38), being opposed to the flow-regulating plate (72) in the axial direction, and
   the flow-regulating plate (72) has the plurality of tapered holes (43) arranged in a central portion in the radial direction and a plurality of cylindrical through holes (74) arranged in a peripheral portion in the radial direction.

4. The fluid sterilization device according to claim 2,

   wherein the other-end-side tube member (18) has a plurality of notch grooves (50) in a circumferential direction in a circumferential edge of an opening that opens at the other end in the axial direction,
   wherein the tube body (11) has a cylindrical member (22) which is disposed coaxially with the other-end-side tube member (18) and which defines an annular space in communication with the outflow port (35) on an outer circumference side between itself and an outer circumference of the other-end-side tube member (18),
   wherein a plate-like transmission member (45) through which ultraviolet light is transmitted comes in contact with the circumferential edge of the opening of the other-end-side tube member (18) to close the circumferential edge of the opening, and
   wherein the light source (12, 67) is disposed on the other end side in the axial direction with respect to the plate-like transmission member (45).

5. The fluid sterilization device according to any one of claims 1 to 4,
   wherein, in a case where a thickness of the flow-regulating plate (42, 72) is denoted by t1, the diameters of the upstream side opening (44a) and the downstream side opening (44b) as the openings on the one end side and the other end side in the axial direction, respectively, in the tapered holes (43), are denoted by di and do, and Ra denotes a predetermined value below 1, a relationship below is satisfied:

$$(2 \cdot tl) / (di + do) < Ra.$$

**Patentansprüche**

1. Strömungsmittelsterilisierungsvorrichtung (10), die Folgendes aufweist:
einen Rohrkörper (11) mit einem rohrförmigen Durchlass, durch welchen ein Strömungsmittel, das durch einen Einflussanschluss (34) an einer Endseite in einer axialen Richtung hinein fließt, in der axialen Richtung zu einem Ausflussanschluss (35) an der anderen Endseite in der axialen Richtung fließt;

eine Flussregulierungsplatte (42, 72), welche den rohrförmigen Durchlass in einem Einlassseitenraum (38), der an der einen Endseite angeordnet ist, und in welchem das Strömungsmittel, das von dem Einflussanschluss (34) hereinfließt, eingeleitet wird, und einen Sterilisierungsraum (39) an der anderen Endseite aufteilt, und die eine Vielzahl von Durchgangslöchern (43, 74) hat, die in axialer Richtung dort hindurch verlaufen; und
eine Lichtquelle (12, 67), welche den Sterilisierungsraum mit ultraviolettem Licht bestrahlt;
wobei die Vielzahl von Durchgangslöchern (43) eine Vielzahl von verjüngten Löchern (43) aufweist, deren Durchmesser sich von der einen Endseite zur anderen Endseite in der axialen Richtung vergrößern,
**dadurch gekennzeichnet, dass**
jedes der Vielzahl von verjüngten Löchern (43) eine Öffnung (44a) der stromaufwärts gelegenen Seite an einer Endseite und eine Öffnung (44b) der stromabwärts gelegenen Seite an der anderen Endseite hat, und wobei die Seitenwand der verjüngten Löcher (43) einen vorbestimmten Verjüngungswinkel (θ) bezüglich der axialen Richtung bildet, und
wobei jedes der Vielzahl von verjüngten Löchern (43) so geformt ist, dass ein Durchmesser der Öffnung (44a) der stromaufwärts gelegenen Seite kleiner ist als ein Durchmesser der Öffnung (44b) der stromabwärts gelegenen Seite, und wobei der vorbestimmte Verjüngungswinkel (θ) ein Winkel ist, mit welchem Strömungsmittel, welche in den Sterilisierungsraum (39) durch die verjüngten Löcher (43) ausgestoßen werden, die benachbart zueinander sind, in Kontakt miteinander kommen, um eine Diffusion bzw. Vermischung in einer radialen Richtung zu unterdrücken.

2. Strömungsmittelsterilisierungsvorrichtung nach Anspruch 1,
wobei der Rohrkörper (11) Folgendes hat:

ein Rohrelement (17) der einen Endseite, welches den Einlassseitenraum (38) an einer Innenumfangsseite definiert; und
ein Rohrelement (18) der anderen Endseite, welches den Sterilisierungsraum (39) an der Innenumfangsseite definiert, welches mit dem Rohrelement (17) der einen Endseite in der axialen Richtung verbunden ist, und welches eine Umfangskante der Flussregulierungsplatte (42, 72) in der axialen Richtung an den Innenumfang eines Verbindungsteils (19) durch eine ringförmige Nut (20) einklemmt, die zwischen ihm selbst und dem Rohrelement (17) der anderen Endseite geformt ist.

3. Strömungsmittelsterilisierungsvorrichtung nach Anspruch 1 oder 2,

wobei der Einflussanschluss (34) sich zum Einlassseitenraum (38) öffnet, wobei er in der axialen Richtung entgegengesetzt zur Flussregulierungsplatte (72) ist, und
wobei die Flussregulierungsplatte (72) die Vielzahl von verjüngten Löchern (43) hat, die in einem mittigen Teil in der radialen Richtung angeordnet sind, und eine Vielzahl von zylindrischen Durchgangslöchern (74), die an einem Umfangsteil in der radialen Richtung angeordnet sind.

4. Strömungsmittelsterilisierungsvorrichtung nach Anspruch 2,

wobei das Rohrelement (18) der anderen Endseite eine Vielzahl von Vertiefungsnuten (50) in einer Umfangsrichtung in einer Umfangskante einer Öffnung hat, die sich am anderen Ende in der axialen Richtung öffnet,
wobei der Rohrkörper (11) ein zylindrisches Element (22) hat, welches koaxial mit dem Rohrelement (18) der anderen Endseite angeordnet ist und welches einen ringförmigen Raum in Verbindung mit dem Ausflussanschluss (35) an einer Außenumfangsseite zwischen sich und einem Außenumfang des Rohrelementes (18) der anderen Endseite definiert,
wobei ein plattenartiges Durchlasselement (45), durch welches ultraviolettes Licht durchgelassen wird, in Kontakt mit der Außenumfangskante der Öffnung des Rohrelementes (18) der anderen Endseite kommt, um die Umfangskante der Öffnung zu schließen, und

wobei die Lichtquelle (12, 67) an der anderen Endseite in der axialen Richtung bezüglich des plattenartigen Durchlasselementes (45) angeordnet ist.

5. Strömungsmittelsterilisierungsvorrichtung nach einem der Ansprüche 1 bis 4,
wobei in einem Fall, wo eine Dicke der Flussregulierungsplatte (42, 72) mit t1 bezeichnet wird, die Durchmesser der Öffnung (44a) der stromaufwärts gelegenen Seite und der Öffnung (44b) der stromabwärts gelegenen Seite als die jeweiligen Öffnungen an der einen Endseite und der anderen Endseite in der axialen Richtung in den verjüngten Löchern (43) mit di und do bezeichnet werden, und wobei Ra einen vorbestimmten Wert unter 1 bezeichnet, eine unten dargestellte Beziehung erfüllt ist:

$$(2 \times t1) / (di + do) < Ra.$$

**Revendications**

1. Dispositif de stérilisation de fluide (10) comprenant :

un corps de tube (11) ayant un passage tubulaire à travers lequel un fluide s'écoulant depuis un orifice d'admission (34) sur un côté d'une première extrémité dans une direction axiale s'écoule dans la direction axiale vers un orifice d'échappement (35) sur le côté d'une autre extrémité dans la direction axiale ;
une plaque de régulation de flux (42, 72) qui divise le passage tubulaire en un espace côté introduction (38) qui est situé sur le côté de la première extrémité et dans lequel le fluide qui s'écoule depuis l'orifice d'admission (34) est introduit, et un espace de stérilisation (39) du côté de l'autre extrémité, et qui a une pluralité de trous traversant (43, 74) qui pénètrent dans la direction axiale ; et
une source de lumière (12, 67) qui irradie l'espace de stérilisation (39) avec de la lumière ultraviolette ;
dans lequel la pluralité de trous traversant (43) comporte une pluralité de trous coniques (43) dont les diamètres augmentent depuis le côté de la première extrémité vers le côté de l'autre extrémité dans la direction axiale,
**caractérisé en ce que**
chacun des trous traversant de la pluralité (43) a une ouverture côté amont (44a) sur le côté de la première extrémité et une ouverture côté aval (44b) sur le côté de l'autre extrémité et dans lequel la paroi latérale des trous coniques (43) forme un angle de cône prédéterminé (θ) par rapport à la direction axiale, et
dans lequel chacun des trous traversant de la pluralité (43) est formé de sorte d'un diamètre de l'ouverture côté amont (44a) est plus petite qu'un diamètre de l'ouverture côté aval (44b), et l'angle de cône prédéterminé (θ) est un angle avec lequel les fluides éjectés dans l'espace de stérilisation (39) à travers les trous coniques (43) qui sont adjacents les uns aux autres viennent en contact les uns avec les autres pour éliminer la diffusion dans la direction radiale.

2. Dispositif de stérilisation de fluide selon la revendication 1,
dans lequel le corps du tube (11) a :

un élément de tube du côté de la première extrémité (17) qui définit l'espace d'introduction (38) sur un côté circonférentiel intérieur ; et
un élément de tube du côté de l'autre extrémité (18) qui définit l'espace de stérilisation (39) sur le côté circonférentiel intérieur, qui est connecté à l'élément de tube du côté de la première extrémité (17) dans la direction axiale, et qui pince un bord circonférentiel de la plaque de régulation de flux (42, 72) dans la direction axiale au niveau de la circonférence intérieure d'une partie de connexion (19) par une rainure annulaire (20) formée entre lui-même et l'élément de tube du côté de la première extrémité (17).

3. Dispositif de stérilisation de fluide selon la revendication 1 ou 2,

dans lequel l'orifice d'admission (34) s'ouvre sur l'espace d'introduction (38), en étant opposé à la plaque de régulation de flux (72) dans la direction axiale, et
la plaque de régulation de débit (72) a une pluralité de trous coniques (43) disposés dans une partie centrale dans la direction radiale et une pluralité de trous traversants cylindriques (74) disposés dans une partie périphérique dans la direction radiale

4. Dispositif de stérilisation de fluide selon la revendication 2,

dans lequel l'élément de tube du côté de l'autre extrémité (18) a une pluralité de rainures d'encoche (50) dans une direction circonférentielle dans un bord circonférentiel d'une ouverture qui s'ouvre au niveau de l'autre extrémité dans la direction axiale,

dans lequel le corps du tube (11) a un élément cylindrique (22) qui est disposé coaxialement à l'élément du tube du côté de l'autre extrémité (18) et qui définit un espace annulaire en communication avec l'orifice d'échappement (35) sur un côté de la circonférence extérieure entre lui-même et une circonférence extérieure de l'élément de tube du côté de l'autre extrémité (18),

dans lequel un élément de transmission en forme de plaque (45) à travers lequel la lumière ultraviolette est transmise entre en contact avec le bord circonférentiel de l'ouverture de l'élément de tube du côté de l'autre extrémité (18) pour fermer le bord circonférentiel de l'ouverture, et

dans lequel la source de lumière (12, 67) est disposée sur le côté de l'autre extrémité dans la direction axiale par rapport à l'élément de transmission en forme de plaque (45).

5. Dispositif de stérilisation de fluide selon l'une quelconque des revendications 1 à 4,
dans lequel, dans le cas où l'épaisseur de la plaque de régulation de flux (42, 72) est désignée par t1, les diamètres de l'ouverture côté amont (44a) et de l'ouverture côté aval (44b) en tant que les ouvertures du côté de la première extrémité et du côté de l'autre extrémité dans la direction axiale, respectivement, dans les trous coniques (43), sont désignés par di et do, et où Ra désigne une valeur prédéterminée inférieure à 1, la relation suivante est satisfaite :

$$(2 \cdot t1)/(di + do) < Ra.$$

FIG. 1A

FIG. 1B

EP 4 292 614 B1

FIG. 2

FIG. 3

12

## FIG. 4A

## FIG. 4B

## FIG. 5

## FIG. 6A

## FIG. 6B

## FIG. 6C

## FIG. 6D

FIG. 7A

FIG. 7B

FIG. 7C

FIG. 7D

FIG. 8

FIG. 9

**EP 4 292 614 B1**

**Patent documents cited in the description**

- JP 2020092856 A

- CN 108793532 A